# EUROPEAN PATENT APPLICATION

(11) **EP 0 523 776 A2**
(43) Date of publication of application: **20.01.1993**
(21) Application number: 92202023.5
(22) Date of filing: 03.07.1992
(51) Int. Cl.: A61K 7/16

(54) **Oral compositions containing a phosphopeptide**

(30) Priority: 17.07.1991 US 731592
(71) Applicant: UNILEVER N.V., NL-3013 AL Rotterdam (NL); UNILEVER PLC, London EC4P 4BQ (GB)
(72) Inventor: Burger, Allan Robert, Edgewater, NJ 07020 (US); Elliott, David Leroy, Edgewater, NJ 07020 (US); Schick, Laura Ann, Edgewater, NJ 07020 (US)
(74) Representative: van Gent, Jan Paulus

(57) **Abstract**

Oral compositions are provided containing a stabilized phosphopeptide. The phosphopeptide is selected from phosphopeptides having 5 to 30 aminoacids and having a particular amino acid sequence. The phosphopeptide can be derived from naturally occurring materials, e.g. from casein. The phosphopeptide is stabilized by the presence in the compositions of an anionic polymer, such as a sulfonate polymer or a carboxylate polymer, preferably a polyacrylate and/or acrylate/maleate copolymer.

## Description

### BACKGROUND OF THE INVENTION

### Field of the Invention

The invention relates to oral compositions, particularly to oral compositions containing a stabilized phosphopeptide.

### Related Art

It is known to include a phosphopeptide or a salt thereof in oral compositions. U.S. Patent 4,933,173 (Bristow et al.) discloses an anticaries composition containing a water-soluble casein material and a particulate hydroxyapatite. International Patent Application WO 82/03008, discloses compositions for inhibiting caries and gingivitis containing polyphosphopeptides. European Patent Application 0 166 055 discloses anticaries compositions containing a digest of a protein or a polypeptide. European Patent Application 0 268 663 discloses oligopeptides of particular sequence as anticaries actives. None of the references mentions any compositions containing an anionic polymer in conjunction with the phosphopeptide.

Unfortunately, the phosphopeptide is unstable and its activity is decreased or lost in the presence of saliva. Thus, an oral composition containing the phosphopeptide and which is stable in the presence of saliva is desirable.

Oral compositions containing anionic polymers, especially polycarboxylates, as anticalculus agents have been disclosed. For example, U.S. Patent 4,842,847 (Amjad) discloses anticalculus compositions based on carboxylic homo- and copolymers and additionally containing fluoride ion and a dental abrasive which may be an inorganic phosphate salt. Similar disclosures are found in U.S. Patent 4,892,725 (Amjad) and U.S. Patent 4,892,724 (Amjad).

U.S. Patent 4,661,341 (Benedict et al.) discloses the use of low molecular weight polyacrylic acids (MW range 3500 to 7500) in dental compositions. U.S. Patent 3,429,963 (Shedlovsky) teaches use of maleate-containing copolymers and vinyl sulfonate polymers in toothpaste. U.S. Patent 4,183,914 (Gaffar et al.) reports use of polymaleates as anticalculus agents. U.S. Patent 4,362,713 (Buck) discloses the use of certain hydrophilic alkali metal and ammonium salts of 1:1 copolymers of styrene and maleic acid and 1:1 copolymers of certain linear 1-alkenes and maleic acid as antiplaque agents.

It has also been disclosed that certain anionic polymers inhibit the enzymatic hydrolysis of polyphosphate salts to orthophosphates. U.S. Patent 4,915,937 (Amjad) discloses oral compositions containing a fluoride source, a dental abrasive, a polyphosphate and an antihydrolysis agent selected from homopolymers of substituted acrylamides, homopolymers of unsaturated sulfonic acids and salts thereof and homopolymers and copolymers of cationic monomers. The antihydrolysis agent is said to inhibit hydrolysis of polyphosphates to orthophosphates.

Gaffar et al., U.S. Patent 4,806,342, U.S. Patent 4,806,340, U.S. Patent 4,808,400, U.S. Patent 4,808,401 disclose a combination of a fluoride ion-providing source and a synthetic linear polymeric polycarboxylate for the inhibition of enzymatic hydrolysis in saliva of a linear molecularly dehydrated polyphosphate salt. Generally suitable synthetic linear polycarboxylates listed in the Gaffar et al. patents include olefinically or ethylenically unsaturated carboxylic acids, the list including acrylic and maleic acids. The synthetic linear polycarboxylate of the Gaffar et al. patents is exemplified by Gantrez S-97^{**(**}^{R**)**} (ex GAF Corporation), a copolymer (1:1) of maleic acid and methoxyethylene.

U.S. Patent 4,534,881 (Sikes et al.) discloses synthetically derived amino acid polymers for the inhibition or retardation of inorganic scaling, such as calcium carbonate deposition in pipes, boilers and the like. The synthetic polymers may contain phosphorylated amino acids.

U.S. Patent 4,866,161 (Sikes et al.) and U.S. Patent 4,868,287 (Sikes et al.) disclose a synthetic polyamino acid compound and a method of inhibiting tartar deposition on teeth by applying the synthetic polyamino acid compound, wherein the anionic amino acids are clustered on one end of the amino acid chain and the nonpolar amino acids are clustered on the other end. The anionic amino acids are independently selected from phosphoserine, phosphohomoserine, phosphotyrosine, phosphothreonine, glutamic acid and aspartic acid. Although the polyamino acids taught by Sikes et al. must be synthesized, Sikes et al. disclose that the presence of phosphoserine residues in natural proteins significantly enhances antitartar activity and illustrate that statherin (a natural salivary phosphoprotein) is an effective tartar inhibitor. Sikes et al. disclose that when phosphopeptides are employed, it is contemplated to use phosphatase inhibitors in conjunction with the phosphopeptide to prevent or inhibit dephosphorylation of the phosphopeptides. Sikes et al. list suitable phosphatase inhibitors including vinyl ether/maleic acid polymers (Gantrez^{**(**}^{R**)**}).

European Patent Application 0 391 629 (Sikes) discloses synthetic polypeptide materials having a formula poly(X)ₘ(Y)ₙ, where each X is independently aspartate, glutamate, glutamine, asparagine, or phosphoserine, each Y independently is a phosphorylated amino acid such as phosphoserine, phosphohomoserine, phosphotyrosine and phosphothreonine; m is 2 to 150, n is 1 to 3, and n + m is greater than or equal to 5.

The poly amino acids taught by Sikes et al. in all of the references discussed above must be synthesized. While statherin, a naturally occurring salivary protein, is mentioned by Sikes et al., it is hard to obtain a commercially sufficient supply of statherin. In the present invention, selected phosphopeptides, which differ from the materials disclosed by Sikes et al. and also differ from statherin, can be obtained from naturally occurring materials (e.g., milk protein), yet can be obtained in amounts sufficient to satisfy commercial demand.

Accordingly, it is an object of the invention to provide oral compositions containing a selected phosphopeptide and a stabilizer which inhibits the phosphopeptide's destabilization in the oral environment.

This and other objects of the invention will become more apparent from the detailed description and examples which follow.

### SUMMARY OF THE INVENTION

The invention includes oral compositions containing an active agent which is a phosphopeptide, a phosphopeptide salt, or mixtures thereof and an anionic polymeric stabilizer for the active agent. The phosphopeptides which are suitable in the present invention contain from 5 to 30 amino acids including an amino acid sequence

A-B-C-D-E

where A, B, C, D and E are independently phosphoserine, phosphothreonine, phosphotyrosine, phosphohistidine, glutamic acid and aspartic acid.

According to the invention, anionic polymers which exhibit a stabilizing effect on a phosphopeptide are selected from the group consisting of carboxylate polymers, sulfonate polymers, polymers having both a carboxylate and a sulfonate moiety, and mixtures thereof. The phosphopeptide's destabilization in the oral environment is decreased due to the presence of the anionic polymer.

A variety of carboxylate and sulfonate polymers were found to be effective according to the present invention. The polymers may contain hypophosphite moieties. Typically, the molecular weight of the anionic polymers employed in the inventive compositions ranges from 500 to 700,000.

### DETAILED DESCRIPTION OF THE INVENTION

Compositions according to the present invention include a phosphopeptide and/or a salt thereof as an essential active ingredient. The phosphopeptides according to the invention contain from 5 to 30 amino acids including an amino acid sequence

A-B-C-D-E

where A, B, C, D and E are independently phosphoserine, phosphothreonine, phosphotyrosine, phosphohistidine, glutamic acid and aspartic acid.

Preferred phosphopeptides are those wherein A, B, and C are independently phosphoserine, phosphothreonine, phosphotyrosine, and phosphohistidine and D and E are independently phosphoserine, phosphothreonine, glutamate and aspartate.

Particularly preferred phosphopeptides are those where A, B and C are phosphoserine and D and E are glutamate.

A mixture of phosphopeptides and/or their salts may be used in the compositions of the present invention. When mixtures are used, it is preferred that those containing the preferred sequence A-B-C-D-E above predominate.

The phosphopeptides or mixture of phosphopeptides is preferably substantially pure at least to the extent of not containing unpalatable impurities.

The phosphopeptides identified as SEQ ID NO:1, SEQ ID NO:2, SEQ ID NO:3, SEQ ID NO:4, and SEQ ID NO:5 in the "Sequence Listing" hereinbelow have been found to be particularly useful in the compositions of the present invention.

The preferred phosphopeptide may be made synthetically by chemical synthesis or genetic engineering or can be extracted from naturally occurring material e.g. milk.

Because of cost considerations, it is currently more economical to extract the phosphopeptide from casein or caseinates and in particular from alpha-s casein or beta-casein. Additionally, there is an increasing consumer demand for products containing ingredients derived from naturally occurring materials. Thus, in the preferred embodiment of the invention, the phosphopeptide is extracted from the naturally occurring material.

Phosvitin may also be used as a source of phosphopeptides. Further, phosphoproteins in cereals, nuts and vegetables, particularly in bran husks or sheaths, may be used to produce phosphopeptides. In particular, rice, wheat, oat, barley or rye brans are suitable sources of phosphopeptides. Soybean and meat contain phosphoproteins which may also be of use in obtaining phosphopeptides.

Casein, in particular alpha-s casein or beta-casein, or salts thereof such as sodium caseinate contain polypeptides which can be cleaved to simpler peptides. Such cleavage may be effected by digestion, such digestion may be chemical, or enzymatic with proteolytic enzymes.

It is preferred to digest casein with a proteolytic enzyme such as trypsin, pepsin, chymotrypsin, papain, thermolysin or pronase. Trypsin is the preferred enzyme. The digested casein is then fractionated into phosphopeptides containing the sequence A-B-C-D-E, and other peptides. Particularly preferred in the compositions of the present invention are the phosphopeptides of SEQ ID NO:1 and SEQ ID NO:2 mentioned above, as well as mixtures of SEQ ID NO:1 and SEQ ID NO:2, and mixtures rich in phosphopetides having the sequence of SEQ ID NO:1 and/or SEQ ID NO:2. Typically, the casein digest contains a mixture of oligopetides of SEQ ID NO:1, SEQ ID NO:2, SEQ ID NO:3, SEQ ID NO:4, and SEQ ID NO:5 as well as other oligopeptides.

The phosphopeptides may be used as such, or in the form of their alkali metal, alkaline earth metal, ammonium or transition metal salts. Typical examples are the sodium salts, the calcium salts, the zinc, copper, stannous, potassium, strontium and magnesium salts. Sodium salts are most preferred. The above phosphopeptides are more fully described in EP 268,663.

The second essential ingredient of the inventive compositions is an anionic polymer. Suitable polymers include carboxylate polymers, sulfonate polymers, polymers having both sulfonate and carboxylate groups, and mixtures thereof. The anionic polymers according to the present invention are linear synthetic polymers. The polymers may be employed in an acid form, or they may be partially or preferably fully neutralized. Suitable salts include alkali metal, such as potassium and, preferably, sodium salts or ammonium salts.

It was found that the stabilization benefit provided by a carboxylate polymer is independent of molecular weight. For ease of formulation, the molecular weight of the polymers ranges from 500 to 700,000, preferably from 1000 to 500,000, most preferably from 1000 to 5000.

The carboxylate polymers suitable for use in the present compositions are described by Gaffar et al., U.S. Patent 4,808,400, which is incorporated by reference herein. Examples of carboxylic acid monomers which may be polymerized to obtain the polymeric carboxylate of the present invention include acrylic, maleic, methacrylic, ethacrylic, alpha-chloroacrylic, crotonic, betacryloxy propionic, sorbic, alpha-chlorosorbic, cinnamic, beta-styrylacrylic, muconic, itaconic, citraconic, mesaconic, glutaconic, aconitic, alphaphenylacrylic, 2-benzyl acrylic, 2-cyclohexylacrylic, angelic, umbellic, fumaric acids and anhydrides. Homopolymers and copolymers of the above acids may be employed.

The carboxylate polymer included in the present invention may also be a copolymer of the above acids with olefinic monomers, for example, vinyl acetate, vinyl chloride, dimethyl maleate, styrene, isobutylene, ethyl vinyl ether, and methyl vinyl ether.

The carboxylate polymer according to the invention may contain phosphinate units. For example, copolymers of acrylic acid and maleic acid (and other carboxylic monomers) containing mono- or disubstituted hypophosphite units along the polymer backbone are effective stabilizers of the phosphopeptide in the oral environment. The latter polymers are described more fully in a co-pending Application Serial Number 510,651, now allowed, incorporated by reference herein.

The preferred carboxylate polymers according to the present invention are polyacrylates and acrylate/maleate copolymers, the polyacrylates being most preferred in view of their performance. The stabilization benefit provided by the carboxylate polymers appears to increase with increasing acrylate content.

The sulfonate polymers suitable for use in the inventive compositions are derived from sulfonate-containing monomers which may be polymerized to obtain the polymeric sulfonate. Examples of operable monomers include, but are not limited to, styrene-sulfonic acids, 2-acrylamido-2-methylalkanesulfonic acids, vinylsulfonic acids, allyloxy hydroxy alkanesulfonic acids, sulfo lower alkyl acrylates, and the like. Homopolymers of sulfonated monomers and their copolymers with other monomers may be employed. Suitable olefinic monomers include, for example, vinyl acetate, vinyl chloride, dimethyl maleate, styrene, isobutylene, ethyl vinyl ether, and methyl vinyl ether.

The preferred sulfonate polymers according to the present invention are homopolymers and copolymers derived from monomers such as styrenesulfonic acid, vinylsulfonic acid, and 2-acrylamido-2-methylpropanesulfonic acid. Commercial examples of such materials are the Versa TL resins (polystyrenesulfonates ex National Starch and Chemical Corporation) and HSP-1180 (homopolymer of 2-acrylamido-2-methylpropanesulfonic acid ex Henkel Corporation).

Polymers containing combinations of carboxylate and sulfonate monomers may also be used as the second essential ingredient of the inventive compositions. Examples of such polymers include but are not limited to copolymers of styrenesulfonic acids and maleic acid (e.g. Narlex D-72^{**(**}^{R**)**} and Versa TL-3^{**(**}^{R**)**} from National Starch and Chemical Corporation), copolymers of acrylic acid and 2-acrylamido-2-methylpropanesulfonic acid, and copolymers of acrylic acid with vinylsulfonic acid (e.g. Narlex LD-52^{**(**}^{R**)**} from National Starch and Chemical Corporation).

A copolymer of acrylic acid (AA) and 2-acrylamido-2-methylpropanesulfonic acid (AMPS) can be prepared by free radical polymerization. Deionized water is used as the solvent medium, and the monomers are placed in the reaction vessel at the desired ratio (for example, a 4:1 molar ratio of AA:AMPS); the concentration of the monomers in the water should be about 25-40% by weight. The polymerization is initiated using either potassium or sodium persulfate initiator at weight ratios (monomer:initiator) of 100:1. Isopropanol can be added at the desired level to limit molecular weight. The polymerization is conducted at 70°C with stirring for 1-2 hours. Additional initiator is added and the temperature is raised to 95°C for 0.5 hours to achieve substantially 100% conversion. After cooling to room temperature, the solution pH is adjusted to about 7 to neutralize the polymer. A similar copolymer, containing about 10% AMPS and 90% acrylic acid, is available under the trade name, Belclene 400^{**(**}^{R**)**}, from Ciba-Geigy Corporation.

The compositions of the present invention are useful as oral compositions and may be used whenever it is desired to deliver phosphopeptides into oral cavity. The compositions may contain from 0.01% to 20% phosphopeptide, preferably from about 0.1 to about 10%, most preferably from about 0.1 to about 5%.

The anionic polymer is employed in the present invention in the amount effective to stabilize a phosphopeptide in the presence of saliva. The amount of polymer will vary and depends on the particular polymer employed, as well as the amount of the phosphopeptide to be stabilized. The optimum amount may be ascertained by employing the experimental procedure outlined in the Examples below. Increased amount of the anionic polymeric stabilizer will result in an increased stabilization of the phosphopeptide. Typically, the amount of the anionic polymeric stabilizer in the inventive compositions varies from about 0.01 to-about 10%, preferably, in view of cost considerations, from about 0.1 to about 5%, most preferably from about 0.1 to 2.5%.

The preferred oral compositions of the present invention are in the form of toothpaste creams, or gels, or mouthwashes. Ingredients typically included in toothpastes and gels may be used in toothpaste and gel compositions in accordance with the invention. Suitable ingredients include abrasive polishing materials, sudsing agents, flavoring agents, humectants, binders, sweetening agents, and water.

Abrasives which may be used in the compositions of the invention include alumina and hydrates thereof, such as alpha alumina trihydrate, magnesium trisilicate, magnesium carbonate, aluminosilicates, such as calcined aluminum silicate and aluminum silicate, calcium carbonate, zirconium silicate, polymethyl methacrylate, powdered polyethylene, silica xerogels, hydrogels and aerogels and the like. Also suitable as abrasive agents are calcium pyrophosphate, insoluble sodium metaphosphate, calcium carbonate, dicalcium orthophosphate, particulate hydroxyapatite and the like. Depending on the form which the oral composition is to take, the abrasive may be present in an amount of from 0 to 70% by weight, preferably 1 to 70% by weight, more preferably from 10 to 70% by weight, particularly for toothpastes.

Humectants contemplated for use in the present invention include glycerol, polyol, sorbitol, polyethylene glycols, propylene glycol, hydrogenated partially hydrolyzed polysaccharides and the like. The humectants are generally present in amounts of from 0 to 80%, preferably 5 to 70% by weight, particularly for toothpastes. Thickeners suitable for use in the invention include silica. Thickeners may be present in toothpaste creams and gels at 0.1 to 20% by weight.

Binders suitable for use in the compositions of the invention include hydroxyethyl cellulose (Natrosol^{**(**}^{R**)**}), sodium carboxymethyl cellulose and hydroxypropyl cellulose (Klucel^{**(**}^{R**)**}), as well as xanthan gums, Irish moss and gum tragacanth. Binders may be present in the toothpaste of the invention to the extent of from 0.01 to 10%. Sweeteners suitable for use in the present dentifrice, preferably at levels of about 0.1% to 5%, include saccharin.

Fluoride sources used in toothpastes such as sodium fluoride, stannous fluoride, sodium monofluorophosphate, zinc ammonium fluoride, tin ammonium fluoride, calcium fluoride and cobalt ammonium fluoride may be, and preferably are, included for delivering anticaries benefit. It has been found in the present invention that addition of fluoride provides additional stabilization of phosphopeptides. Preferred compositions of the invention include the fluoride source. Fluoride ions are typically provided at a level of from 0 to 1500 ppm, preferably 50 to 1500 ppm, although higher levels up to about 3000 ppm may be used.

Surfactants, such as a soap, anionic, nonionic, cationic, amphoteric and/or zwitterionic, may be present within the range of 0 to 15%, preferably 0.1 to 15%, more preferably 0.25 to 10% by weight. Anionic surfactants are most preferred, such as sodium dodecyl sulfate, sodium lauryl sarcosinate and sodium dodecylbenzene sulfonate.

Flavors are usually included in toothpastes in low amounts, such as from 0.01 to about 5% by weight, especially from 0.1% to 5%.

Ingredients mentioned above as suitable for toothpastes are generally suitable for gels, as will be apparent to one of skill in the art of toothpaste and gel formulation. Thus, except where otherwise noted, references to toothpastes are to be construed as applying to gels as well.

Typically, mouthwashes comprise a water/alcohol solution, flavor, humectant, sweetener, sudsing agent, and colorant. The corresponding compounds mentioned above which are used in toothpastes, are generally suitable within the ranges above for mouthwashes as well. The mouthwash can include ethanol at a level of from 0 to 60%, preferably from 5 to 30% by weight.

Dyes/colorants suitable for dentifrices, i.e., FD&C Blue #1, FD&C Yellow #10, FD&C Red #40, etc., may be employed in the dentifrices of the invention.

Various other optional ingredients may be included in the compositions of the invention, such as preservatives, vitamins such as vitamin C and E, other antiplaque agents such as stannous salts, copper salts, strontium salts and magnesium salts. Also included may be pH adjusting agents, anticaries agents such as urea, calcium glycerophosphate, anti-staining compounds such as silicone polymers, plant extracts, desensitizing agents for sensitive teeth such as potassium nitrate and potassium citrate, and mixtures thereof.

The following specific examples further illustrate the invention, but the invention is not limited thereto.

### Preparation of a sodium salt of casein phosphopeptide

A tryptic digest of casein (Hyprol 8052^{**(**}^{R**)**}) was obtained from Quest International Corporation. A 10% aqueous solution of the digest was prepared and the pH of the resulting solution was adjusted to pH 4.8 by the addition of concentrated hydrochloric acid. The solution was then centrifuged to separate any undigested casein. The supernatant was then made up to 1% calcium chloride, and an equal volume of ethanol was added. The precipitate was collected by centrifugation and freeze dried to yield the dry calcium salt of casein phosphopeptide.

The sodium salt was then prepared by a cation exchange method, as follows.

2 grams of Dowex^{**(**}^{R**)**} resin (50WX8, 50-100 mesh, acid form) were added to a 50 ml clear centrifuge tube. The resin was washed three times with 50 ml of water. Subsequently, 40 ml of cold (4°C) 2.5% calcium casein phosphopeptide (clarified by centrifugation) was added to the tube. After gentle agitation in a tube rotator for 30 minutes at 4°C, the acidic supernatant (pH about 2.7) was decanted and neutralized to pH 7.0 with 1.5 ml of 2N NaOH. Calcium content in the supernatant was measured by Atomic Absorption Spectroscopy, and it was found that 99.5% calcium was removed. The resulting solution was freeze-dried to obtain the dry sodium salt of casein phosphopeptide.

The resulting sodium salt of casein phosphopeptide contained phosphopeptides of SEQ ID NO:1, SEQ ID NO:2 and other casein phosphopeptides.

### Experimental Method

Saliva sediment was isolated from parafilm stimulated saliva by centrifugation, resuspended in a small volume of distilled deionized water, and centrifuged again. The resulting washed sediment was then resuspended in 0.2M sodium acetate buffer, pH 5.7, to obtain a 50% (w/v) saliva sediment solution.

Test solutions consisting of 2% phosphopeptide (sodium salt) and an anionic polymer at the desired concentration were also prepared in the pH 5.7 buffer. Equal volumes (100 µL) of saliva sediment solution and a test solution were mixed to obtain a final concentration of 1% phosphopeptide, 25% saliva sediment in each sample, and the desired content (as indicated in the Examples) of an anionic polymeric stabilizer. A control sample contained 1% phosphopeptide and 25% saliva sediment and no stabilizer.

All samples were incubated at 37°C with mixing for 24 hours. Destabilization of a phosphopeptide is directly proportional to the loss of bound phosphorus content of the phosphopeptide. The phosphopeptide's destabilization in each sample was determined by the following method.

### Measurement of Loss of Bound Phosphorus

The following method is a modification of the method by Chen, et. al. Analytical Chemistry 28, 1756 (1956).

Reagent: A mixture of 1 part 2.5% ammonium molybdate with 1 part 6N H₂SO₄, 1 part 10% ascorbic acid and 2 parts distilled deionized water.

20 µl of a phosphopeptide-containing sample prepared as described above was diluted to 4 ml with distilled deionized water to obtain a test sample. 4 ml of the reagent was added to the test sample. Samples were incubated at 60°C for 30 minutes, then cooled to room temperature.

Absorbance at 820 nm was determined (Bausch and Lomb Spectronic 88, 15 mm path length). Absolute free phosphorus content could be determined by constructing a standard curve using KH₂PO₄.

In the present Examples, relative % loss of bound phosphorus values were calculated by assuming 100% loss of bound phosphorus for the control sample which contained a phosphopeptide and did not contain a stabilizer, since it was found that different pools of saliva sediment would destabilize the phosphopeptide to somewhat different degrees. The results in the Examples indicate qualitative trend in stabilization. It should be noted, however, that the absolute value of the phosphopeptide's loss of bound phosphorus in the absence of a stabilizer was always about 100%.

### EXAMPLE I

A control sample and samples containing various carboxylate polymers were prepared as described above. All samples, except the control sample, contained 1% of a polymeric carboxylate stabilizer.

The results that were obtained are summarized in Table I.

**Table I**

| Sample | Carboxylate Polymer | Acrylate: Maleate | Monomer: Hypophosphite | MW | Loss of Bound Phosphorus % |
|---|---|---|---|---|---|
| Control | None | | | | 100 |
| A | ¹Acrysol A-5^{**(**}^{R**)**} | 1:0 | 1:0 | 500,000 | 9 |
| B | ²Acrysol LMW-10N^{**(**}^{R**)**} | 1:0 | 1:0 | 1,000 | 14 |
| C | ³Belsperse 161^{**(**}^{R**)**} | 1:0 | 16:1 | 3,500 | 2 |
| D | ⁴AM-A | 1:0 | 16:1 | 2,800 | 44 |
| E | ⁵AM-B | 1:0 | 8:1 | 1,550 | 46 |
| F | ⁶AM-C | 1.5:1 | 8:1 | 1,200 | 33 |
| G | ⁷AM-F | 4:1 | 8:1 | 1,400 | 36 |
| H | ⁸Belclene 201^{**(**}^{R**)**} | 0:1 | 1:0 | about 800 | 64 |
| I | ⁹Gantrez S-97^{**(**}^{R**)**} | -- | -- | 700,000 | 24 |

| | | | | | |
|---|---|---|---|---|---|
| ¹Obtained from Rohm and Haas | | | | | |
| ²Obtained from Rohm and Haas | | | | | |
| ³Obtained from Ciba-Geigy | | | | | |
| ^{4,5,6,7} Prepared as described in a co-pending Application Serial No. 510,651, now allowed. | | | | | |
| ⁸Obtained from Ciba-Geigy | | | | | |
| ⁹A copolymer (1:1) of maleic acid and methoxyethylene obtained from GAF corporation. | | | | | |

The example illustrates that in the compositions including a phosphopeptide and a carboxylate polymer according to the invention, the phosphopeptide's destabilization in the presence of saliva was reduced or, even, almost prevented.

The example illustrates that at the concentration of 1%, the mixture of Belsperse 161^{**(**}^{R**)**} and a phosphopeptide exhibited the best stabilization. Next to Belsperse, Acrysol LMW-10N^{**(**}^{R**)**} and Acrysol A-5^{**(**}^{R**)**} also resulted in particularly effective stabilization of phosphopeptide.

Among the acrylate and acrylate/maleate polymers in Table I, the polymer which did not include any acrylate (Belclene 201^{**(**}^{R**)**}) was least effective, although significant stabilization was still achieved.

### EXAMPLE II

Samples containing 1% phosphopeptide (sodium salt) and a polymeric carboxylate (Belsperse 161^{**(**}^{R**)**}) were prepared according to the experimental procedure described above. The phosphopeptide's destabilization was measured as described above. The results that were obtained are summarized in Table II.

**Table II**

| % Carboxylate Polymer | Loss of Bound Phosphorus (%) |
|---|---|
| 0.00 | 100.0 |
| 0.25 | 20.1 |
| 0.50 | 10.0 |
| 1.00 | 2.1 |

The example illustrates that the addition of the polymeric carboxylate, even at small concentrations, stabilized the phosphopeptide. Increased level of addition of the polymeric carboxylate resulted in the increased stabilization of the phosphopeptide.

### EXAMPLE III

Samples containing 1% phosphopeptide (sodium salt) and various anionic polymeric stabilizers were prepared according to the experimental procedure described above. Samples B, C and D contained 1% of a polymeric anionic stabilizer; sample E contained 0.1% of a stabilizer. The phosphopeptide's loss of bound phosphorus was measured as described above. The results that were obtained are summarized in Table III.

**Table III**

| Sample | Polymer | Description | Loss of Bound Phosphorus % |
|---|---|---|---|
| A | None | | 100 |
| B | ¹Narlex D-72^{**(**}^{R**)**} | 1:1 styrenesulfonate/maleate (MW 15,000) | 25 |
| C | ²Versa TL-70^{**(**}^{R**)**} | Polystyrenesulfonate (MW 70,000) | 29 |
| D | ³AMPS/AA | Copolymer containing 80% acrylic acid and 20% 2-acrylamido-2-methylpropanesulfonic acid | 3 |
| E | ³AMPS/AA | Copolymer containing 80% acrylic acid and 20% 2-acrylamido-2-methylpropanesulfonic acid | 37 |

| | | | |
|---|---|---|---|
| ^{1,2}Obtained from National Starch and Chemical Corporation. | | | |
| ³Prepared as described in the specification. | | | |

The example illustrates that sulfonate polymers as well as polymers which have both sulfonate and carboxylate groups effectively decrease or prevent the phosphopeptide's destabilization in the presence of saliva.

### EXAMPLE IV

Carboxylate polymers (Belsperse 161^{**(**}^{R**)**} and Gantrez S-97^{**(**}^{R**)**}) and a sulfonate polymer (Versa TL-70^{**(**}^{R**)**}) were examined for their ability to stabilize a phosphopeptide (1%, sodium salt) in the presence of 25% saliva sediment and in the presence of sodium fluoride. Each polymer was examined at a concentration of 1%. Fluoride was added at 10, 100, 500, and 1000ppm levels. Additionally, stabilization of the phosphopeptide in solutions containing fluoride in the absence of a polymeric carboxylate was ascertained. Samples were prepared and loss of bound phosphorus measurements were conducted as described above. The results that were obtained are summarized in Table IV.

**TABLE IV**

| Loss of Bound Phosphorus (%) in the Presence of | | | | |
|---|---|---|---|---|
| ppm F- | F- Alone | Belsperse 161^{**(**}^{R**)**} | Gantrez S-97^{**(**}^{R**)**} | Versa TL-70^{**(**}^{R**)**} |
| 0 | 100.2 | 9.2 | 38.4 | 20.8 |
| 10 | 103.8 | 4.8 | 30.1 | 16.3 |
| 100 | 73.9 | 1.5 | 26.0 | 14.2 |
| 500 | 53.3 | 1.5 | 17.5 | 10.9 |
| 1000 | 66.5 | 1.3 | 24.4 | 10.4 |

The example illustrates that although fluoride at higher concentrations provided some stabilization of the phosphopeptide, substantial increases in stabilization of the phosphopeptide were achieved by the addition of the anionic polymer, according to the present invention.

### EXAMPLE V

The following agents were tested (at 0.1%, 0.5%, and 1.0%) for their ability to prevent the phosphopeptide's destabilization in the presence of 25% saliva sediment.

| % LOSS OF BOUND PHOSPHORUS | | | |
|---|---|---|---|
| Agent | 0.1% | 0.5% | 1.0% |
| Belsperse 161^{**(**}^{R**)**} | 40.2 | 22.2 | 10.0 |
| Gantrez S-97^{**(**}^{R**)**} | 63.3 | 51.8 | 42.5 |
| Belclene 201^{**(**}^{R**)**} | 73.4 | 63.3 | 45.3 |
| AM-C⁴ | 72.7 | 65.5 | 52.1 |
| AM-A⁴ | 64.0 | 51.8 | 75.4 |
| AM-B⁴ | 69.9 | 79.8 | 70.2 |
| AM-F⁴ | 80.4 | 90.7 | 68.2 |
| Narlex D-72^{**(**}^{R**)**} | 18.7 | 14.6 | 16.2 |
| Versa TL 70^{**(**}^{R**)**} | 31.4 | 27.4 | 26.6 |
| Acrysol LMW-10N^{**(**}^{R**)**} | 52.6 | 35.0 | 17.6 |
| Acrysol A-5^{**(**}^{R**)**} | 47.8 | 39.8 | 31.4 |
| AMPS/AA | 40.5 | 32.2 | 18.7 |
| AM-S⁴ | 21.6 | 5.1 | 13.3 |
| Sokalan CP5² | 56.2 | 62.0 | 49.4 |
| Sokalan CP7³ | 53.7 | 60.6 | 62.1 |

| | | | |
|---|---|---|---|
| ¹Obtained from Rohm and Haas; polymer containing 40% acrylic acid 40% acrylamide, and 20% ethyl acrylate. | | | |
| ²obtained from BASF; 4:1 acrylate/maleate copolymer, MW=70,000. | | | |
| ³Obtained from BASF; 2:1 acrylate/maleate copolymer, MW=50,000. | | | |
| ⁴Prepared as described in a co-pending Application Serial No. 510,651, now allowed. | | | |

The example illustrates clearly that various anionic polymers, within the scope of the invention, prevent the phosphopeptide's destabilization in the presence of saliva.

### EXAMPLE VI

A typical formulation for a mouthwash, containing a stabilized phosphopeptide according to the invention, is as follows:

| MOUTHWASH FORMULATION | |
|---|---|
| INGREDIENTS | % BY WEIGHT OF FINAL COMPOSITION |
| Ethanol | 12.5 |
| 70% Sorbitol | 7 |
| Phosphopeptide (Sodium salt) | 5 |
| Tween 20 | 0.55 |
| Preservatives* | 0.2 |
| Flavor | 0.1 |
| Anionic polymer (as stabilizer for the phosphopeptide) | 0.1 - 5% |
| Dye | <.01 |
| Sodium Saccharinate | .065 |
| Sodium chloride | .05 |
| Na acetate | .015 |
| Acetic acid | .015 |
| H₂0 | to 100 |
| pH | 6.5 |

| | |
|---|---|
| * 0.1% methylparaben | |
| + 0.1% propylparaben | |

### EXAMPLE VII

Typical toothpaste formulations, containing a stabilized phosphopeptide according to the invention, are as follows:

| TOOTHPASTE FORMULATIONS pH 6-8 | | |
|---|---|---|
| | % BY WEIGHT OF FINAL COMPOSITION | |
| | A | B |
| 70% Sorbitol | 64 | 39 |
| Abrasive silica | 10 | 10 |
| Thickening silica | 9 | 10 |
| Phosphopeptide (Na salt) | 5 | 5 |
| Polyethylene glycol (PEG 32^{**(**}^{R**)**}) | 5 | 5 |
| Anionic polymer (as stabilizer for the | | |
| phosphopeptide) | 5 | 5 |
| Sodium Lauryl Sulfate | 1.5 | 1.5 |
| Flavor | 1 | 1 |
| Sodium saccharinate | 0.3 | 0.2 |
| Na Fluoride | 0.24 | 0.24 |
| Preservative (Na Benzoate) | .08 | .08 |
| Dye | < .01 | --- |
| Titanium oxide | --- | 1 |
| Sodium carboxymethyl cellulose | 0.15 | 0.6 |
| H₂0 | to 100 | to 100 |

It should be understood that the specific forms of the invention herein illustrated and described are intended to be representative only. Changes, including but not limited to those suggested in the specification, may be made in the illustrative embodiments without departing from the clear teachings of the disclosure. Accordingly, reference should be made to the following appended claims in determining the full scope of the invention.

## Claims

1. An oral composition comprising:
a) from about 0.01% to about 20% of an ingredient selected from the group consisting of a phosphopeptide, a salt of a phosphopeptide and mixtures thereof, wherein the phosphopeptide contains from 5 to 30 amino acids and includes an amino acid sequence A-B-C-D-E, wherein A, B, C, D and E are independently phosphoserine, phosphothreonine, phosphotyrosine, phosphohistidine, glutamate and aspartate; and
b) an anionic polymeric stabilizer selected from the group consisting of a carboxylate polymer, a sulfonate polymer, a polymer having both a sulfonate and a carboxylate moiety, a carboxylate polymer containing phosphinate units, and mixtures thereof, the stabilizer being present in an amount effective to stabilize the phosphopeptide.

2. The composition of claim 1 wherein the anionic polymeric stabilizer is the carboxylate polymer.

3. The composition of claim 2 wherein the carboxylate polymer is selected from the group consisting of a polyacrylate, an acrylate/maleate copolymer, and mixtures thereof.

4. The composition of claim 1 wherein the anionic polymeric stabilizer has a molecular weight in a range of from 500 to 700,000.

5. The composition of claim 1 wherein the phosphopeptide comprises the sequence A-B-C-D-E wherein A, B and C are each phosphoserine, and D and E are each glutamate.

6. The composition of claim 5 wherein the phosphopeptide comprises a mixture of phosphopeptides having SEQ ID NO:1 and SEQ ID NO:2.

7. The composition of claim 1 wherein the phosphopeptide is a derivative of a naturally occurring material.

8. The composition of claim 7 wherein the naturally occurring material is a casein.

9. The composition of claim 1 wherein the anionic polymeric stabilizer is selected from the group consisting of the sulfonate polymer, the polymer having both a carboxylate and a sulfonate moiety, and mixtures thereof.

10. The composition of claim 1 wherein the composition comprises from about 0.01% to about 10% of the anionic polymeric stabilizer.

11. The composition of claim 1 wherein the composition further comprises a fluoride source present in an effective amount to reduce caries.


SEQUENCE LISTING

(1) GENERAL INFORMATION:

(i) APPLICANT: Burger, Allan R., Elliott, David L.,
Schick, Laura A.

(ii) TITLE OF INVENTION: Oral Compositions Containing a
Phosphopeptide

(iii) NUMBER OF SEQUENCES: 5

(iv) CORRESPONDENCE ADDRESS:

(A) ADDRESSEE: Patent Department, Unilever United
States, Inc.

(B) STREET: 45 River Road

(C) CITY: Edgewater

(D) STATE: New Jersey

(E) COUNTRY: USA

(F) ZIP: 07020

(v) COMPUTER READABLE FORM:

(A) MEDIUM TYPE: Diskette-3.5 inch, 1.44 Mb
storage

(B) COMPUTER: IBM PS/2

(C) OPERATING SYSTEM: MS-DOS, Version 3.30

(2) INFORMATION FOR SEQ. ID NO:1:

(i) SEQUENCE CHARACTERISTICS:

(A) LENGTH: 21

(B) TYPE: Amino acid

(ix) FEATURE:
(A) NAME/KEY: Phosphoserine
(B) LOCATION: 6
(D) OTHER INFORMATION: Post-translationally
phosphorylated serine
(ix) FEATURE:
(A) NAME/KEY: Phosphoserine
(B) LOCATION: 8
(D) OTHER INFORMATION: Post-translationally
phosphorylated serine
(ix) FEATURE:
(A) NAME/KEY: Phosphoserine
(B) LOCATION: 9
(D) OTHER INFORMATION: Post-translationally
phosphorylated serine
(ix) FEATURE:
(A) NAME/KEY: Phosphoserine
(B) LOCATION: 10
(D) OTHER INFORMATION: Post-translationally
phosphorylated serine
(ix) FEATURE:
(A) NAME/KEY: Phosphoserine
(B) LOCATION: 17
(D) OTHER INFORMATION: Post-translationally
phosphorylated serine

(xi) SEQUENCE DESCRIPTION: SEQ ID NO:1:
Glu Met Glu Ala Glu Ser Ile Ser Ser Ser Glu Glu Ile Val Pro Asn
1               5                   10                  15
Ser Val Glu Gln Lys
            20

(2) INFORMATION FOR SEQ. ID NO:2:
(i) SEQUENCE CHARACTERISTICS:
(A) LENGTH: 24
(B) TYPE: Amino acid
(C) STRANDEDNESS:
(D) TOPOLOGY:
(ix) FEATURE:
(A) NAME/KEY: Phosphoserine
(B) LOCATION: 14
(D) OTHER INFORMATION: Post-translationally
phosphorylated serine
(ix) FEATURE:
(A) NAME/KEY: Phosphoserine
(B) LOCATION: 16
(D) OTHER INFORMATION: Post-translationally
phosphorylated serine
(ix) FEATURE:
(A) NAME/KEY: Phosphoserine
(B) LOCATION: 17
(D) OTHER INFORMATION: Post-translationally
phosphorylated serine

(ix) FEATURE:
(A) NAME/KEY: Phosphoserine
(B) LOCATION: 18
(D) OTHER INFORMATION: Post-translationally
phosphorylated serine
(xi) SEQUENCE DESCRIPTION: SEQ ID NO:2:

Glu Leu Glu Glu Leu Asn Val Pro Gly Glu Ile Val Glu Ser Leu Ser
1               5                   10                  15

Ser Ser Glu Glu Ser Ile Thr Arg
            20

(2) INFORMATION FOR SEQ. ID NO:3:
(i) SEQUENCE CHARACTERISTICS:
(A) LENGTH: 20
(B) TYPE: Amino acid
(ix) FEATURE:
(A) NAME/KEY: Phosphoserine
(B) LOCATION: 7
(D) OTHER INFORMATION: Post-translationally
phosphorylated serine
(ix) FEATURE:
(A) NAME/KEY: Phosphoserine
(B) LOCATION: 8
(D) OTHER INFORMATION: Post-translationally
phosphorylated serine

(ix) FEATURE:
(A) NAME/KEY: Phosphoserine
(B) LOCATION: 9
(D) OTHER INFORMATION: Post-translationally
phosphorylated serine
(ix) FEATURE:
(A) NAME/KEY: Phosphoserine
(B) LOCATION: 15
(D) OTHER INFORMATION: Post-translationally
phosphorylated serine
(xi) SEQUENCE DESCRIPTION: SEQ ID NO:3:

Asn Thr Met Glu His Val Ser Ser Ser Glu Glu Ser Ile Ile Ser Gln
1               5                   10                  15

Glu Thr Tyr Lys
            20

(2) INFORMATION FOR SEQ. ID NO:4:
(i) SEQUENCE CHARACTERISTICS:
(A) LENGTH: 25
(B) TYPE: Amino acid
(ix) FEATURE:
(A) NAME/KEY: Phosphoserine
(B) LOCATION: 11
(D) OTHER INFORMATION: Post-translationally
phosphorylated serine

(ix) FEATURE:
(A) NAME/KEY: Phosphoserine
(B) LOCATION: 12
(D) OTHER INFORMATION: Post-translationally
phosphorylated serine
(ix) FEATURE:
(A) NAME/KEY: Phosphoserine
(B) LOCATION: 13
(D) OTHER INFORMATION: Post-translationally
phosphorylated serine
(ix) FEATURE:
(A) NAME/KEY: Phosphoserine
(B) LOCATION: 16
(D) OTHER INFORMATION: Post-translationally
phosphorylated serine
(xi) SEQUENCE DESCRIPTION: SEQ ID NO:4:

Asn Ala Asn Glu Glu Glu Tys Ser Ile Gly Ser Ser Ser Glu Glu Ser
1 5 10 15

Ala Glu Val Ala Thr Glu Glu Val Lys
20 25
(2) INFORMATION FOR SEQ. ID NO:5:
(i) SEQUENCE CHARACTERISTICS:
(A) LENGTH: 11
(B) TYPE: Amino acid

(ix) FEATURE:
(A) NAME/KEY: Phosphoserine
(B) LOCATION: 4
(D) OTHER INFORMATION: Post-translationally
phosphorylated serine
(ix) FEATURE:
(A) NAME/KEY: Phosphothreonine
(B) LOCATION: 5
(D) OTHER INFORMATION: Post-translationally
phosphorylated threonine
(ix) FEATURE:
(A) NAME/KEY: Phosphoserine
(B) LOCATION: 6
(D) OTHER INFORMATION: Post-translationally
phosphorylated serine
(xi) SEQUENCE DESCRIPTION: SEQ ID NO:5:

Glu Gln Leu Ser Thr Ser Glu Glu Asn Ser Lys
1               5                   10